# EUROPEAN PATENT SPECIFICATION

(11) **EP 2 258 396 B1**
(45) Date of publication and mention of the grant of the patent: **06.09.2017**
(21) Application number: 09717697.8
(22) Date of filing: 24.02.2009
(51) Int. Cl.: A61K 9/70, A61K 31/519

(54) **TRANSDERMALLY ABSORBABLE PREPARATION**
TRANSDERMAL ABSORBIERBARES PRÄPARAT
PRÉPARATION POUVANT ÊTRE ABSORBÉE DE FAÇON TRANSDERMIQUE

(30) Priority: 03.03.2008 JP 2008052539
(43) Date of publication of application: 08.12.2010
(73) Proprietor: Hisamitsu Pharmaceutical Co., Inc., Tosu-shi Saga 841-0017 (JP)
(72) Inventor: KURIBAYASHI, Mitsuru, Tsukuba-shi Ibaraki 305-0856 (JP); UCHIDA, Naoyuki, Tsukuba-shi Ibaraki 305-0856 (JP)
(74) Representative: Dr. Gassner & Partner mbB
(86) International application number: PCT/JP2009/053267
(87) International publication number: WO 2009/110351

(56) References cited:
- EP-A1- 1 661 560
- EP-A1- 1 857 103
- WO-A1-00/61120
- WO-A1-01/07018
- WO-A1-02/38139
- WO-A1-2005/102393
- WO-A1-2005/115355
- US-A1- 2004 142 024
- US-A1- 2007 184 097

## Description

### [Technical Field]

The present invention relates to a transdermally absorbable preparation comprising a complex of an organic acid and an organic acid salt and a drug.

### [Background Art]

In transdermally absorbable preparations, attempts to improve transdermal absorbability of active ingredients have conventionally been carried out by blending powders of a silicate compound such as silicic anhydride (silica) and an organic acid salt, etc. in a base.

For example, it is reported that by making a patch wherein polyhydric alcohol and silicic anhydride, etc. are blended in an adhesive base, the polyhydric alcohol can be blended in a larger amount and the transdermal absorbability of a drug can be increased by its transdermal-absorption enhancing action, and also an adhesive property can be improved by blending silicic anhydride without lowering the aggregation force of an adhesive matrix even with a large amount of the polyhydric alcohol (ref. Patent Literatures 1 and 2) . In addition, it is reported that by making a transdermally absorbable preparation wherein a powder(silicic anhydride or organic acid salt, etc.) and a drug are blended, bioavailability or sustained-release properties of active ingredients can be enhanced (ref. Patent Literatures 3-7). However, the skin permeability of drugs is not sufficient even in these attempts, and therefore, a further improvement has been desired.

In the meantime, although it is necessary to blend a drug in a high concentration to an adhesive base in order to improve transdermal absorbability, there was a problem that a drug low in solubility to a base is crystallized when blended in the base in a high concentration. For this, suppression of the crystallization of a drug by using silicic anhydride, etc. has been proposed (Patent Literature 8).

However, because the suppression of crystallization (non-crystallization) of a drug by silicic anhydride is easily affected by temperature/humidity of a preserved atmosphere and in fact, crystallization gradually proceeds after preparing a preparation, it is difficult to provide a preparation blended with silicic anhydride as a transdermally absorbable preparation stable over time, and therefore, a further pharmaceutical improvement has been required.

Under such circumstances, Patent Literature 9 discloses that a stable preparation can be obtained with a transdermally absorbable preparation composed of a complex consisting of a silicate compound and an organic acid, and a drug and a base; and some effects have been acknowledged with this preparation.

However, as a result of further investigation by the present inventors, a preparation composed of a complex of a silicate compound and an organic acid and a drug has been shown to generate needle-like crystals of the drug in the preparation depending on the condition of drying process aiming at removal of solvent. In particular in cases of pharmaceuticals, the amount of a residual solvent in a preparation must be decreased below a safety permissible value, and therefore the drying condition in the solvent removal process is strictly set. As a result, the organic acid having a low boiling point volatilizes during the solvent removal process, thereby an amount necessary to maintain the formation of the above complex cannot be blended. Under such conditions, needle-like crystals of the drug are generated in the preparation due to decreased stability of the drug in the base.

Patent Literature 10 discloses a transdermally absorbable preparation of which a base comprises composite particles which are composed of at least a silicate compound, in particular silicic anhydride, an organic acid, in particular an acetic acid, an organic acidsalt, in particular sodium acetate and a basic drug, inparticular risperidone. The preparation can be in the form of a patch. The preparation may comprise the drug in a non-crystallized form.

Patent Literature 11 discloses a transdermally patch comprising a melting point lowering agent and an adhesive base. The melting point lowering agent maybe acetic acid or sodium acetate. The drug may be a basic drug such as risperidone.

Thus, further enhancement of the stability of preparations has been desired.
Patent Literature 1: JP 3027018
Patent Literature 2: JP A 8-27003
Patent Literature 3: JP A 5-271056
Patent Literature 4: JP A 2004-502725
Patent Literature 5: JP A 9-505554
Patent Literature 6: JP A 4-312525
Patent Literature 7: JP A 11-302161
Patent Literature 8: JP 3526864
Patent Literature 9: JP A 2005-054033
Patent Literature 10: EP 1 857 103 A1
Patent Literature 11: EP 1 661 560 A1

### [Summary of Invention]

### Technical Problem

Therefore, an object of the present invention is to provide a transdermally absorbable preparation that, even when a drug with poor solubility in a base is added in a high concentration, is stable over time and can suppress crystallization of the drug, excelling in transdermal absorbability.

### Solution to Problem

During extensive research to solve the above problems, the inventors have found that the transdermally absorbable preparation comprising a complex of an organic acid and an organic acid salt has better stability than transdermally absorbable preparations comprising a complex of a silicate compound and an organic acid, and further found that the particle diameter of the complex is important in drug crystallization, and the inventors have accomplished the invention.

Namely, the present invention provides a transdermally absorbable preparation comprising, in its base, at least a complex of acetic acid and sodium acetate, and a drug. The drug is risperidone.

The preparation has peaks at one or more positions from 13.7 ± 0.2°, 22.5 ± 0.2°, 34.9 ± 0.2° (2θ) in its X-ray diffraction pattern and is absent an X-ray peak at 9° (2θ).

50% or more of the complexes have a particle diameter of 1-30 µm.

The present invention also provides the above transdermally absorbable preparation, characterized in that the preparation further comprises a silicate compound.

The present invention also provides the above transdermally absorbable preparation, characterized in that the silicate compound is silicic anhydride.

The present invention also provides the above transdermally absorbable preparation, characterized in that the specific surface area of the silicic anhydride is 100 m²/g or more.

The present invention provides the above transdermally absorbable preparation, wherein the preparation has peaks at one or more positions from 11.2 ± 0.2°, 13.7 ± 0.2°, 21.0 ± 0.2°, 22.5 ± 0.2°, 25.1 ± 0.2°, 34.9 ± 0.2°, and 41.8 ± 0.2° (2θ) in the X-ray diffraction pattern and is absence of X-ray peak at 9° (2θ).

In addition, the present invention provides the above transdermally absorbable preparation, characterized in that the preparation is a patch having a support with an adhesive layer on its one side.

### [Advantageous Effects of Invention]

With the present invention, a transdermally absorbable preparation is made to comprise a complex of an organic acid and an organic acid salt and a drug, thereby making it possible to blend in the preparation even a high concentration of a drug that has a low solubility in a base and that is apt to crystallize; accordingly, a preparation having an action to suppress crystallization can be provided.

### [Brief Description of Drawings]

[Fig. 1] Figure 1 is a graph showing results of X-ray diffraction of a mixture of acetic acid and sodium acetate.
[Fig. 2] Figure 2 is a graph showing results of X-ray diffraction of sodium acetate alone.
[Fig. 3] Figure 3 is a micrograph of a preparation comprising complex particles of acetic acid and sodium acetate.

### [Best embodiment of invention]

The transdermally absorbable preparation of the present invention is not particularly limited as long as the preparation is for transdermal administration, and includes patches such as plasters and gel patches, creams, plasters, lotions, ointments, sprays, etc.

Among them, patches having a support and an adhesive layer at least on one side of the support are preferably used due to their absorbability and easiness in administration.

Therefore, in the present invention, patches are sometimes particularly described in detail, but the invention is not limited thereto.

In the base of the transdermally absorbable preparation of the present invention, in particular in its adhesive layer when the preparation is a patch, at least a complex of an organic acid and an organic acid salt and a drug are comprised.

In the present invention, at least the above organic acid and the organic acid salt form a complex in the preparation. The complex in the present invention refers to a powder wherein complexed ingredients are physically and/or chemically adsorbed on the surface of particulates; and this complex may be further complexed by adsorption of other ingredients such as drugs, or silicate compounds (including cases in which a complex of an organic acid and an organic acid salt is adsorbed by other ingredients).

Crystallization of a drug is suppressed by complexation of organic acid and organic acid salt. Suppression of crystallization of the drug in the base is enabled by inclusion of the complexed organic acid and organic acid salt (a complex) and a drug into the base (an adhesive layer in the case of patches) of the transdermally absorbable preparation, compared to those without complexation of organic acid and organic acid salt.

As the organic acid of the present invention, acetic acid is used.

The blend amount of the organic acid is preferably 0 . 1-5 times of moles relative to 1 mole of the drug, considering stability and skin permeability as the transdermally absorbable preparation, and physical properties of the preparation. This is because when the molar ratio of the organic acid to the drug is too low, there is a tendency that the transdermal absorption is reduced, and when the molar ratio is too high, there is a tendency that physical properties such as aggregation and dispersiveness of the base are reduced.

In addition, in the base of the transdermally absorbable preparation (or in the adhesive layer when the preparation is a patch), the concentration of the organic acid is 0.01-50 mass%, preferably 0.05-30 mass%, and more preferably 0.1-20 mass%. When the concentration of the organic acid is too low, skin permeability tends to decrease, and when the concentration is too high, physical properties of the base tend to decrease and skin irritation tends to occur.

The organic acid salt that is used in the invention is sodium acetate.

The blend amount of the organic acid salt is preferably 0.1-5 times of moles relative to 1 mole of the drug, more preferably 0.5-5 times of moles, and furthermore preferably 1-5 times of moles. The blend amount of the organic acid salt affects the residual amount of the organic acid in the preparation. Both improvement in skin permeability and suppression of crystallization can be achieved by making the residual amount of the organic acid in the preparation close to a predetermined amount.

In the present invention, for example when acetic acid is used as the above organic acid and sodium acetate is used as the organic acid salt, an X-ray diffraction pattern of the base is characterized by having peaks at one or more positions from 13.7 ± 0.2°, 22.5 ± 0.2° and 34.9 ± 0.2° (2θ). When the base has peaks at such positions, a complex of acetic acid and sodium acetate is contained in the base.

Here, X-ray diffraction in the present invention was measured using a powder X-ray diffractometer X' Pert Pro (Spectris Co., Ltd.), under the condition with an X-ray tube (voltage 45 kV, current 40 mA), scan angle (5°-50°), and scan rate (0.05°-2°/min).

Moreover, in the present invention, as the particle diameter of the complex in the preparations decreases, crystallization of the drug can be suppressed more efficiently. Typically, preparations are prepared so that 50% or more, preferably 70% or more, and more preferably 80% or more of the complexes contained in the preparation have a particle diameter of 1 µm-30 µm, preferably 1 µm-25 µm, more preferably 1 µm-20 µm.

Measurement of such particle diameters can be carried out by microscopic observation.

Furthermore, in the present invention, a silicate compound can be contained in the preparation in addition to the above organic acid, the organic acid salt, and the drug. By comprising the silicate compound, skin permeability of the transdermally absorbable preparation can be improved.

Examples of the silicate compound include silicic anhydride, calcium silicate, magnesium silicate, aluminum silicate, aluminum magnesium silicate, magnesium silicate aluminate, sodium magnesium silicate, and the like; among these, silicic anhydride is particularly preferable. In addition, as for silicic anhydride, although the one without any treatment on the surface of particulates (hydrophilic) and the one with a lipophilic treatment on the surface are commercially available, the one without any treatment (hydrophilic) is more preferable. Further, as for silicic anhydride, the one with a small particle diameter and large specific surface area is more preferable; for example, the one in which the particle diameter is 7-40 nm, is commercially available and can preferably be used. Although the specific surface area of silicic anhydride is not particularly limited, it is preferably 100 m²/g or larger, more preferably 300 m²/g or larger.

These silicate compounds are preferably blended with an amount of 0.5-20 wt% based on the weight of the base (in cases of a patch, the weight of the adhesive layer) of the transdermally absorbable preparation, more preferably 2-10 wt%, particularly preferably 3-5 wt%. This is because when the blend amount of the silicate compound is too small, skin permeability tends to decrease over time, and in the meantime, when the blend amount is too large, a decrease in drug releasability occurs, resulting in a tendency of decreased skin permeability.

The drug may be used in a free basic form, in its pharmaceutically acceptable acid addition salt form, or in combination of these. The drug is blended preferably in 3-30 wt% based on the weight of the base of the transdermally absorbable preparation in view of physical properties of the preparation and the transdermal absorbability, more preferably in 5-20 wt%, particularly preferably in 10-20 wt%.

Furthermore, in the present invention, when acetic acid is used as an organic acid, sodium acetate as an organic acid salt, risperidone as a drug and silicic anhydride as another ingredient, it is possible to complex these four ingredients, or to complex acetic acid, sodium acetate and risperidone. The effects identical to those of the present invention can be obtained through any of such complexation.

In the transdermally absorbable preparation of the present invention, in addition to the above ingredients, other bases may be appropriately selected depending on the dosage form of the preparation; in the case when a complex of an organic acid and an organic acid salt, and a drug and a silicate compound are contained in the base, or the complex thereof is contained in the base, the base is preferably a lipophilic base. This is because when a lipophilic base is used, the crystallization-suppression effect of the complex of organic acid and organic acid salt can be maintained without any reduction of the effect.

Examples of the lipophilic base used in the present invention include, in particular in cases that the transdermally absorbable preparation is a patch, as a base for the adhesive layer, preferably Vaseline, styrene/isoprene/styrene block copolymer, acrylic acid ester adhesive base, polyisobutylene, and polydimethylsiloxane.

As an acrylic adhesive base, a polymer or a copolymer of (meth)acrylic acid ester and/or a copolymer of the above (meth)acrylic acid alkyl ester and other functional monomers are preferably used.

As a rubber adhesive base, the followings are preferably used: natural rubber, synthetic rubber, styrene/isoprene/styrene block copolymer (SIS), isoprene rubber, polyisobutylene (PIB), styrene/butadiene/styrene block copolymer (SBS), styrene-butadiene rubber (SBR), and polybutene.

As a silicone adhesive base, those having polydimethylsiloxane, etc. as a main ingredient are available.

Among the adhesive base, SIS and acrylic acid ester copolymer are particularly preferred. These adhesive bases may be used in one kind alone or two or more kinds in combination. The blend amount of these adhesive bases is preferably 5-80 wt% based on the weight of the entire composition of the adhesive layer, and more preferably 10-60 wt%, and particularly preferably 20-50 wt%, considering the formation of the adhesive layer and the tissue permeability of active ingredients.

Further, in addition to the above base, a tackifying resin may be added according to adhesiveness . As usable tackifying resins, examples include rosin derivatives (e.g., rosin, glycerin esters of rosin, hydrogenated rosin, glycerin esters of hydrogenated rosin, pentaerythritol esters of rosin and the like), alicyclic saturated hydrocarbon resins (e.g., Arkon P -100, Arakawa Chemical Industries, Co., Ltd.), aliphatic hydrocarbon resins (e.g., Quintone B170, Zeon Corporation), terpene resins (e.g., Clearon P-125, Yasuhara Chemical), maleic acid resins and the like. Among these tackifying resins, in particular, glycerin esters of hydrogenated rosin, alicyclic saturated hydrocarbon resins, aliphatic hydrocarbon resins and terpene resins are preferred. These tackifying resins may be used in one kind alone or two or more kinds in combination.

Further, a plasticizer may be blended in the above adhesive base. As plasticizers which can be used in the invention, examples include petroleum oils (e.g., paraffin type process oil, and the like), squalane, squalene, vegetable oils (e.g., olive oil, camellia oil, castor oil, tall oil, peanut oil), dibasic acid esters (e.g., adipate esters and the like), liquefied rubber (e.g., polybutene, liquefied isoprene rubber), diethylene glycol, polyethylene glycol, glycol salicylate, propylene glycol, dipropylene glycol, crotamiton and the like. Among these, in particular, liquid paraffin, liquefied polybutene, glycol salicylate, and crotamiton are preferable.

In addition, in a case that the dosage form is an ointment, a non-aqueous base such as Vaseline is used, and in addition to the above plasticizers, higher fatty acids such as myristic acid or its esters, waxes such as whale wax, surfactants such as polyethylene are preferably blended.

In the transdermally absorbable preparation of the invention, a transdermal absorption enhancer may further be blended appropriately. As the absorption enhancer used in the invention, examples include fatty alcohols such as isostearyl alcohol, fatty acids such as capric acid, fatty acid derivatives such as propylene glycol monolaurate and isopropyl myristate, propylene glycol, polyethylene glycol, diethanolamine laurate, and the like; among these, propylene glycol monolaurate, isostearyl alcohol, diethanolamine laurate, isopropyl myristate and capric acid are preferably used. These transdermal absorption enhancers may be used in one kind alone or in combination of two or more kinds. In addition, the blend amount of the transdermal absorption enhancer is preferably 1-30 wt%, more preferably 2-20 wt%, and particularly preferably 3-10 wt%, in consideration of sufficient permeability of active ingredients to the skin as a preparation and skin irritation and the like.

In addition, if necessary, other agents such as antioxidant, filler, cross-linking agent, preservative, ultraviolet absorber and dissolving agent may be blended in the transdermally absorbable preparation of the invention.

The transdermally absorbable preparation of the invention can be obtained by forming a complex through mixing an organic acid and an organic acid salt. Such a complex can be obtained by mixing sodium acetate, acetic acid, a drug, and other substances if necessary.

The patches according to the invention can be prepared by any known method. For example, an acetic acid, sodium acetate and risperidone, and a silicate compound if necessary are mixed, the mixture is dissolved or suspended in a solvent such as dichloromethane, toluene, hexane or ethyl acetate together with an adhesive base ingredient, then spread on a releasable liner or a support and the solvent is removed by drying, then the resultant is attached to a support or a releasable liner to obtain the preparation.

When the transdermally absorbable preparation of the invention is an ointment, it can be prepared by any known method. For example, a silicate compound, acetic acid, risperidone, sodium acetate, a higher fatty acid ester, waxes, a surfactant, and hydrocarbons are heated or dissolved by heating and added, mixed homogenously by a paddle mixer, then cooled to room temperature while stirring, to obtain an ointment.

In the following, the invention is explained in more detail by test examples and examples; however, dosage forms and formulation are not limited to these examples. In addition, various modifications are possible within the technical idea of the invention.

### [Examples]

### 1. Preparation of transdermally absorbable preparation comprising a complex of acetic acid and sodium acetate

### (1) Preparation of sodium acetate

From sodium acetate (mean particle diameter (50% median): 367 µm), sodium acetate preparations having the following four kinds of particle diameter distribution were obtained.

**[Table 1]**

| | No. | 1 | 2 | 3 | 4 |
|---|---|---|---|---|---|
| Particle diameter (µm) | 10% diameter | 8.8 | 10.0 | 23.0 | 41.3 |
| | 50% diameter (median diameter) | 28.1 | 37.8 | 50.7 | 87.5 |
| | 90% diameter | 85.1 | 69.0 | 90.1 | 174.9 |
| | mv: volume mean diameter | 40.7 | 39.9 | 55.0 | 100.3 |
| | mn: number mean diameter | 7.4 | 5.8 | 6.9 | 38.1 |
| | ma: area mean diameter | 19.7 | 22.6 | 36.2 | 73.3 |
| | cs: specific surface area | 0.3 | 0.3 | 0.2 | 0.1 |
| | std: standard deviation | 28.7 | 22.7 | 24.7 | 47.4 |

### (2) Preparation of transdermally absorbable preparation

3.2% of the sodium acetate having one of the four kinds of particle diameter prepared as above, 3.5% of acetic acid, 8.0% of risperidone, and 3.0% of silicic acid are mixed; to the resulting mixture, an adhesive base ingredient comprising 12% of styrene/isoprene/styrene block copolymer (SIS), 47% of alicyclic saturated hydrocarbon resin, 16.3% of liquid paraffin, and 7% of propylene glycol monolaurate was added, and the entire composition was dissolved or suspended in toluene, applied on a releasing sheet and the solvent was removed by drying; then it was attached to a support, giving a matrix patch preparation having a different particle diameter of the complex (plaster thickness: 100 µm) (Examples 1-4). In addition, using sodium acetate having a mean particle diameter (50% median) of 367 µm, a patch preparation was prepared similarly (Comparative example 1). Here, the particle diameter of the complex can be controlled by the condition of preparation.

### 2. Structural analysis by X-ray diffraction

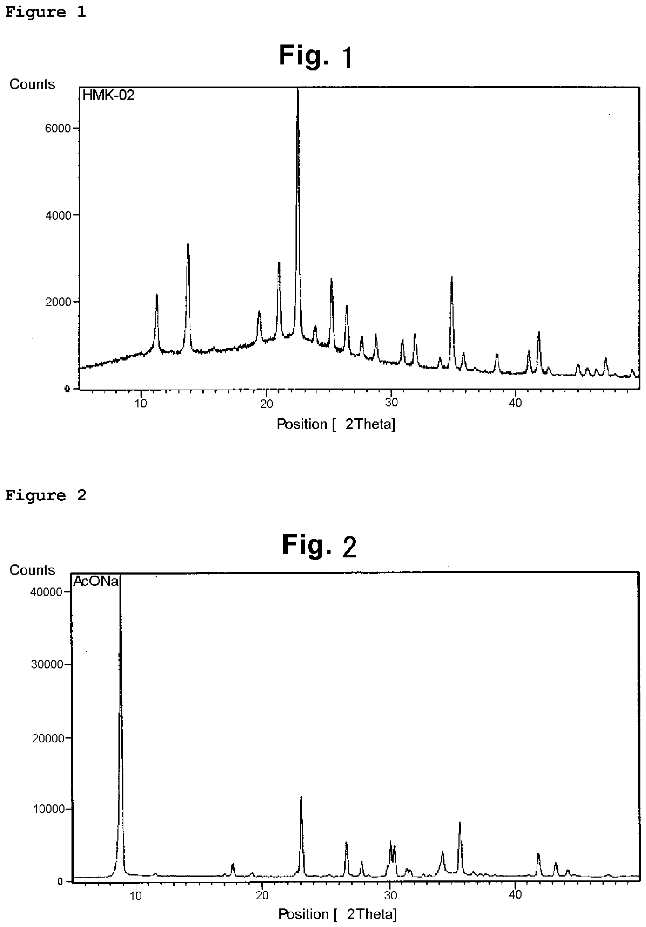

(A) The patch of Example 1 prepared by the above method was subjected to X-ray diffraction using an X-ray diffractometer X'Pert Pro (Spectris Co., Ltd.), under the condition with an X-ray tube (voltage 45 kV, current 40 mA), scan angle (5°-50°), and scan rate (0 . 05°-2°/min) . Table 2 shows the results.
(B) Sodium acetate (particle diameter (50% medidan) of 28.1 µm), acetic acid, and a silicate compound were mixed and subjected to X-ray diffraction under the same condition as in (A) . Figure 1 shows the results.
(C) Sodium acetate (particle diameter (50% median) of 28. 1 µm) alone was subjected to X-ray diffraction under the same condition as in (A). Figure 2 shows the results.

**[Table 2]**

| Powder X-ray diffraction peak (Example 1) | | |
|---|---|---|
| 2θ (degree), | d (Å) | Strength (cps) |
| 11.12 | 7.95 | 1645.37 |
| 13.64 | 6.49 | 2672.05 |
| 19.73 | 4.50 | 374.98 |
| 20.90 | 4.25 | 2273.4 |
| 22.36 | 3.97 | 5280.42 |
| 23.72 | 3.75 | 353.22 |
| 25.03 | 3.55 | 1515.19 |
| 26.29 | 3.39 | 947.37 |
| 27.48 | 3.24 | 755.88 |
| 28.61 | 3.12 | 558.26 |
| 30.77 | 2.90 | 673.41 |
| 31.81 | 2.81 | 750.7 |
| 33.62 | 2.66 | 576.63 |
| 34.76 | 2.58 | 2121.66 |
| 35.68 | 2.51 | 424.33 |
| 38.38 | 2.34 | 452.87 |
| 40.91 | 2.20 | 612.15 |
| 41.72 | 2.16 | 1087.53 |
| 42.55 | 2.12 | 200.29 |
| 44.91 | 2.02 | 248.9 |
| 45.62 | 1.99 | 313.18 |
| 46.38 | 1.96 | 197.19 |
| 47.10 | 1.93 | 489.99 |
| 49.28 | 1.85 | 232.66 |

The results clarified that the mixture of acetic acid and sodium acetate shows a crystal structure having characteristic peaks at 13.7±0.2°, 22.5±0.2°, and 34.9±0.2° (2θ) (Fig. 1) . This suggests the formation of complex particles of acetic acid/sodium acetate. In contrast to this, when the complexation is incomplete, a peak characteristic to sodium acetate is observed at 9° (2θ) (Fig. 2). Furthermore, from the result of the X-ray diffraction of Example 1 shown in Table 2, in the inventive preparation, complexation is considered to be completed and complex particles are sufficiently formed.

### 3. Microscopic observation of complex-particle diameter

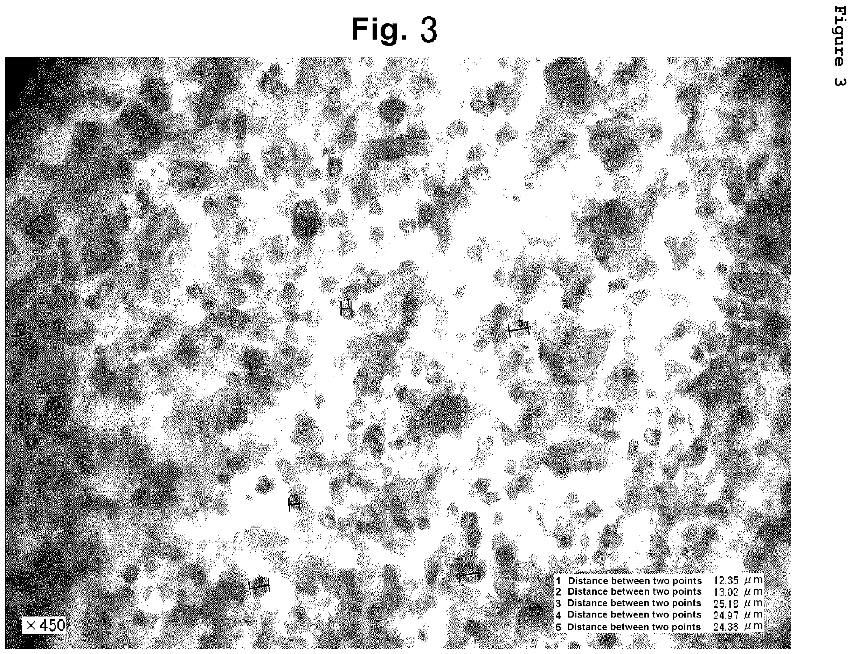

Complex particles of acetic acid/sodium acetate in the preparation (Example 1), that was prepared in accordance with the above procedure 1 and showed no drug crystallization, were observed. As a result, it was found that the preparation showing no drug crystallization comprises complexes having a particle diameter of 1-30 µm in its base, and that many complexes have a particle diameter of 10-25 µm, and approximately 50% of the complexes have a particle diameter of 1-30 µm (Fig. 3).

### 4. Blend amount of sodium acetate, skin permeability and preparation stability

Sodium acetate (particle diameter (50% median) of 28.1 µm) was used with the various blend amounts shown in Examples 5-7 in Table 3 below, and transdermally absorbable preparations were prepared in accordance with the above method.

As Comparative example 2, a transdermally absorbable preparation without addition of sodium acetate was prepared similarly. Here, an adhesive base was added such that the ingredients and compositional ratio identical to Example 1 were contained with the entire preparation (including the amount of adhesive base) being 100%.

**[Table 3]**

| | Composition | Residual amount of acetic acid | Skin permeability | Presence/ absence of drug crystal |
|---|---|---|---|---|
| Comparative example 2 | Drug: 10% | 1.91% (1.30 times moles) | 18.41 | Large amount of crystals |
| | SiA:: 3% | | | |
| | AA: 4.4% | | | |
| | +Adhesive base | | | |
| Example 5 | Drug: 10% | 3.56% (2.43 times moles) | 40.53 | Slight amount of crystals |
| | SiA: 3% | | | |
| | AA: 4.4% | | | |
| | SoA: 2.0% | | | |
| | +Adhesive base | | | |
| Example 6 | Drug: 10% | 3.65% (2.49 times moles) | 47.51 | No crystal |
| | SiA: 3% | | | |
| | AA: 4.4% | | | |
| | SoA: 3.0% | | | |
| | +Adhesive base | | | |
| Example 7 | Drug: 10% | 4.14% (2.82 times moles) | 54.80 | No crystal |
| | SiA: 3% | | | |
| | AA: 4.4% | | | |
| | SoA: 4.0% | | | |
| | +Adhesive base | | | |

| | | | | |
|---|---|---|---|---|
| SiA: Silicic acid, AA: Acetic acid, SoA: Sodium acetate | | | | |

By blending sodium acetate, it has become clear that the skin permeability significantly improves and drug crystallization is effectively suppressed. In addition, it was confirmed that the blend amount of sodium acetate affects the residual amount of acetic acid in the preparations . As the residual amount of acetic acid increases, improvement effect of skin permeability and suppressing effect of drug crystallization are enhanced.

### 5. Stability of preparation

The preparation comprising a complex of acetic acid and sodium acetate is stable, and has no drug crystals or has only a slight amount of them. Furthermore, evaluation of the complexation of acetic acid and sodium acetate in the preparation by means of presence/absence of X-ray peaks shows no peaks characteristic to sodium acetate, indicating that sodium acetate used has been completely complexed.

On the other hand, the preparation of Comparative example 1 shows a large amount of drug crystals (Table 4).

**[Table 4]**

| | Complex particles in the preparation | | Stability | Crystal | Degree of complexation (presence/ absence of X-ray peaks of sodium acetate) |
|---|---|---|---|---|---|
| | presence/ absence of 1-30 µm particles | Percentage of 1-30 µm particles | | | |
| Comparative example 1 | Absent | - | Poor | Large amount | Present |
| Example 1 | Present | 50% or more | Good | Absent | Absent |
| Example 2 | Present | 50% or more | Good | Absent | Absent |
| Example 3 | Present | 50% or more | Good | Absent | Absent |
| Example 4 | Present | 50% or more | Fairly good | Slight amount | Slight peaks are present |

### 6. Adhesive property of preparation

When crystals are generated on the surface of the adhesive layer of a preparation, it is generally known that the contact area between the skin and the adhesive layer decreases, resulting in a decrease in adhesiveness of the preparation.

Adhesive property of the preparations was measured as follows . Test pieces (each with a diameter of 15 mm) of the preparation showing no drug crystal (Example 1) and the preparation showing drug crystal (Comparative example 1) were attached to a ring load (20 g), set on a probe tack tester with a digital counter (No. 1216S, Rigaku Corporation), and measurement was performed with a rate of pulling of 5 cm/s and a contact time of 1 s. As the test probe, a bakelite cylinder type (φ 5 mm) was used. Table 5 shows the results.

In the preparation without drug crystal showed a significant improvement in the probe adhesiveness, compared to the preparation having crystals. Therefore, generation of no drug crystal in the preparations is important not only for increasing the stability, but also for increasing adhesiveness of the transdermally absorbable preparations. By using the complex of organic acid and organic acid salt of the present invention, it becomes possible to prepare preparations that do not generate drug crystals.

**[Table 5]**

| | Presence/absence of drug crystal | Probe adherence property (gF/φ5mm, N=3) |
|---|---|---|
| Comparative example 1 | Present | 351.7±25.5 |
| Example 1 | Absent | 542.7±5.7 |

## Claims

1. A transdermally absorbable preparation comprising, in its base, a complex of acetic acid and sodium acetate, and a drug,
wherein the preparation has peaks at one or more positions from 13.7±0.2°, 22.5±0.2°, 34.9±0.2°(2θ) in its X-ray diffraction pattern and is absent an X-ray peak at 9° (2θ), wherein 50% or more of the complexes have a particle diameter of 1-30 µm, and
wherein the drug is risperidone.

2. The transdermally absorbable preparation according to Claim 1, further comprising a silicate compound.

3. The transdermally absorbable preparation according to Claim 2, wherein the silicate compound is silicic anhydride.

4. The transdermally absorbable preparation according to Claim 3, wherein the specific surface area of the silicic anhydride is 100 m²/g or more.

5. The transdermally absorbable preparation according to any one of Claims 1 to 4, wherein the preparation has peaks at one or more positions from 11.2±0.2°, 13.7±0.2°, 21.0±0.2°, 22.5±0.2°, 25.1±0.2°, 34.9±0.2°, 41.8±0.2°(2θ) in its X-ray diffraction pattern and is absence of X-ray peak at 9° (2θ).

6. The transdermally absorbable preparation according to any one of Claims 1 to 5, wherein the preparation is a patch having a support with an adhesive layer on its one side.

## Patentansprüche

1. Transdermal absorbierbares Präparat, welches in seiner Basis einen Komplex aus Essigsäure und Natriumacetat und ein Arzneimittel umfasst, wobei das Präparat in seinem Röntgendiffraktionsmuster Spitzen an einer oder mehreren Positionen aus 13,7±0,2°, 22,5±0,2°, 34,9±0,2° (2θ) aufweist und keine Röntgenstrahlungsspitze bei 9° (2θ) aufweist, wobei 50% oder mehr der Komplexe einen Partikeldurchmesser von 1-30 µm aufweisen und wobei das Arzneimittel Risperidon ist.

2. Transdermal absorbierbares Präparat nach Anspruch 1, welches weiterhin eine Silikatverbindung umfasst.

3. Transdermal absorbierbares Präparat nach Anspruch 2, wobei die Silikatverbindung Kieselsäureanhydrid ist.

4. Transdermal absorbierbares Präparat nach Anspruch 3, wobei die spezifische Oberfläche des Kieselsäureanhydrids 100 m²/g oder mehr beträgt.

5. Transdermal absorbierbares Präparat nach einem der Ansprüche 1 bis 4, wobei das Präparat in seinem Röntgendiffraktionsmuster Spitzen an einer oder mehreren Positionen aus 11,2±0,2°, 13,7±0,2°, 21,0±0,2°, 22,5±0,2°, 25,1±0,2°, 34,9±0,2°, 41,8±0,2° (2θ) aufweist und keine Röntgenstrahlungsspitze bei 9° (2θ) aufweist.

6. Transdermal absorbierbares Präparat nach einem der Ansprüche 1 bis 5, wobei das Präparat ein Pflaster ist, welches auf seiner einen Seite einen Träger mit einer Kleberschicht aufweist.

## Revendications

1. Préparation absorbable par voie transdermique comprenant, dans sa base, un complexe d'acide acétique et d'acétate de sodium, et un médicament,
dans laquelle la préparation a des crêtes à une ou plusieurs positions à partir de 13,7+/-0,2°, 22,5+/-0,2°, 34,9+/-0,2° (2θ) dans son diagramme de diffraction de rayons X et est absente d'une crête de rayons X à 9° (2θ),
dans laquelle 50 % ou plus des complexes ont un diamètre particulaire de 1 à 30 µm, et
dans laquelle le médicament est la rispéridone.

2. Préparation absorbable par voie transdermique selon la revendication 1, comprenant en outre un composé de silicate.

3. Préparation absorbable par voie transdermique selon la revendication 2, dans laquelle le composé de silicate est l'anhydride silicique.

4. Préparation absorbable par voie transdermique selon la revendication 3, dans laquelle la surface spécifique de l'anhydride silicique est de 100 m²/g ou plus.

5. Préparation absorbable par voie transdermique selon l'une quelconque des revendications 1 à 4, dans laquelle la préparation a des crêtes à une ou plusieurs positions à partir de 11,2+/-0,2°, 13,7+/-0,2°, 21,0+/-0,2°, 22,5+/-0,2°, 25,1+/-0,2°, 34,9+/-0,2°, 41,8+/-0,2° (2θ) dans son diagramme de diffraction de rayons X et est absente d'une crête de rayons X à 9° (2θ).

6. Préparation absorbable par voie transdermique selon l'une quelconque des revendications 1 à 5, dans laquelle la préparation est un pansement ayant un support avec une couche adhésive sur un de ses côtés.
